# EUROPEAN PATENT SPECIFICATION

(11) **EP 3 041 814 B1**
(45) Date of publication and mention of the grant of the patent: **08.11.2017**
(21) Application number: 14790356.1
(22) Date of filing: 01.09.2014
(51) Int. Cl.: C07C 29/62

(54) **PROCESS FOR THE PRODUCTION OF DICHLOROHYDRINS**
VERFAHREN ZUR HERSTELLUNG VON DICHLORHYDRINEN
PROCÉDÉ DE PRODUCTION DE DICHLORHYDRINES

(30) Priority: 03.09.2013 IT RM20130488
(43) Date of publication of application: 13.07.2016
(73) Proprietor: Eurochem Engineering S.R.L., 20139 Milano (IT); Conser S.P.A., 00144 Rome (IT)
(72) Inventor: SANTACESARIA, Elio, I-20139 Milano (IT); TESSER, Riccardo, I-81100 Caserta (IT); VITIELLO, Rosa, I-84018 Scafati (SA) (IT); DI SERIO, Martino, I-74013 Cava dei Tirreni (SA) (IT)
(74) Representative: Sarpi, Maurizio
(86) International application number: PCT/IT2014/000227
(87) International publication number: WO 2015/033365

(56) References cited:
- WO-A1-2006/020234

## Description

### Technical field of the invention

This invention is related to the chlorohydrins production process starting from glycerol as feedstock and hydrochloric acid in the presence of an opportune catalyst. In particular, the invention deals with a new process for producing dichlorohydrins with a high selectivity in the production of α,γ-dichlorohydrin from glycerol and hydrochloric acid in the presence of a new class of catalysts constituted by acyl chlorides.

### State of art

Glycerol can be used as raw material for the production of di-chlorohydrins, in particular α,γ-dichlorohydrin, that is an intermediate for producing epichlorohydrin, useful in the production of epoxy resins [1-7].
The overall reaction scheme in the production of α,γ-dichlorohydrin starting from glycerol and hydrochloric acid is the following one:

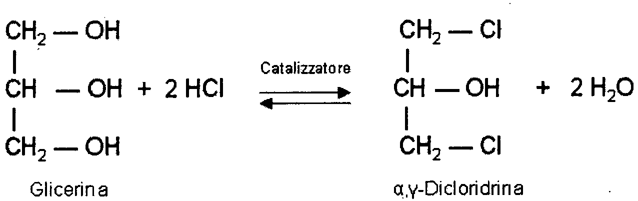

Actually, the reaction proceeds through two consecutive steps giving place in the first step to monochlorohydrins: mainly α-monochloroydrin with small amounts of β-monochloroydrin. Then a second step of chlorination occurs giving place to the desired product α,γ-dichlorohydrin together with small amounts of α,β-dichlorohydrin. A more detailed reaction scheme is reported below: 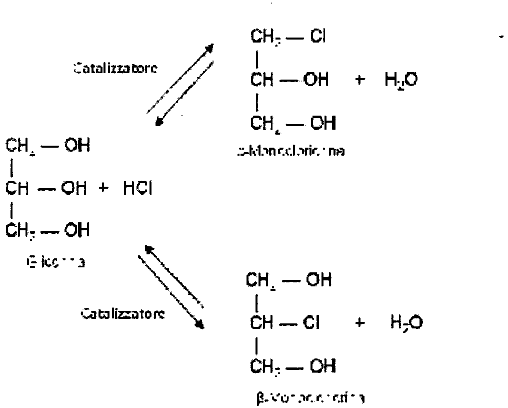 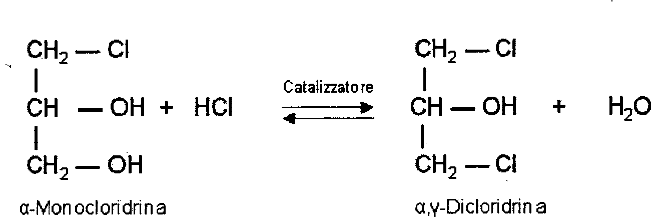 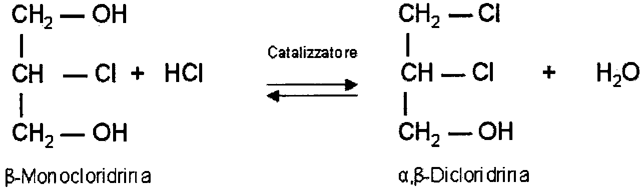 The traditional, very old processes, based on the use of glycerol as raw material proposed the use of aqueous hydrochloric acid and acetic acid as catalyst. The reaction was performed in the temperature range of 80-100°C [8-11].
Some patents describe processes in which an organic solvent, inert to the reaction and immiscibile with water but a good solvent for α,γ-dichlorohydrin, was added. In this case. the boiling temperature of the solvent was the reaction temperature obviously changing with the chosen solvent but never higher than 110°C [12].
Some other patents describe the reaction followed by complicated operations of neutralization, extraction and distillation for recovering α,γ-dichlorohydrin [13,14].
All these processes have some important drawbacks, as:
- the loss of catalyst during the reaction considering the low boiling point of acetic acid (117°C),
- the slowering of the reaction caused by the accumulation of water in the reaction mixture occurring either when aqueous hydrochloric acid is added as reagent or when water is formed as a consequence of the reaction.
- the difficulty of separating the desired product α,γ-dichlorohydrin from the reaction mixture at the end of the reaction.
These drawbacks together with the high cost of glycerol hindered in the past a further development of these processes. At the present time, the most practiced process for producing di-chlorohydrins start use propene as raw material and gives place to a mixture of di-chlorohydrins containing 70 % of α,β- dichlorohydrin and 30 % α,γ-dichlorohydrin. Also this process has some drawbacks. As a matter of fact, the high content α,β-dichlorohydrin is a problem, because, the dehydrochlorination of α,β- dichlorohydrin to epichlorohydrin is much slower than the one of α,γ-dichlorohydrin. This has a negative impact on both the size of the industrial reactors and on the yields [5,6].
Therefore, the reaction using glycerol instead of propene as raw material would be potentially more convenient provided that glycerol is available at low cost. At this purpose, it is useful to remember that the production of biodiesel gives place to 10% b.w. of glycerol as by-product. As a consequence, the glycerol availability increases and the cost decreases. Moreover, considering that the cost of glycerol is strongly affected by the purification it must be pointed out that hydrochlorination can be made also by using unrefined glycerol.

Some patents have recently been published claiming the production of chlorohydrins from glycerol using anhydrous gaseous hydrochloric acid. All these patents suggest the use of carboxylic acids as catalysts promoting the reaction and the difference between the mentioned patents is related to the type of carboxylic acid used and/or for the adopted operative conditions. One of the advantages of producing chlorohydrins from glycerol and gaseous hydrochloric acid in the presence of carboxylic acids as catalysts with respect to the route via propene is the high selectivity to α,γ-dichlorohydrin that is normally obtained. This means the possibility to reduce the volume of the dehydrochlorination reactors for obtaining epichlorohydrin and for the same volume an increase of productivity.
One of the aspects much considered in the different published patents is the adoption of carboxylic acids, as catalyst, less volatile than acetic acid having a boiling point (117°C) near to the reaction temperature. In the patent proposed by Solvay [15], for example, the use of adipic acid is proposed. In the patent published by Eurochem Engineering [16] the use of the following carboxylic acids is proposed: propionic acid, malonic acid, levulinic acid, citric acid, succinic acid and is suggested the possibilità of recovering by stripping α,γ-dichlorohydrin. In the patent published by DOW Global Technologies [17] an exhaustive very long list of carboxylic acids as possible catalysts is reported but in particular the advantage of operating under pressure is stressed being the reaction rate and yields strongly affected by the partial pressure of HCl [18,19]. In the patent by CONSER SpA [20] it has been suggested the opportunità to operate by using two different catalysts in sequence, adding malic acid at low pressure (1-2 bars) initially, adding then succinic acid at higher pressure (8-10 bars).
The performances of the carboxylic acid as catalysts have been intensively studied [18,21,22] and a rough correlation has been found between the pKa of the acid (must be comprised in the range 4-5) and the catalytic activity. For example, the sequence acetic acid, mono-, di- and tri-chloroacetic shows a decreasing activity, because, the acid strength of the mentioned acids increases with the content of chlorine [22]. Some carboxylic acids resulted selective in producing monochlorohydrins [21-23]. A reaction mechanism has been suggested to explain the observed kinetic behaviour [23]. In the frame of the studies performed with the objective to find the optimal catlayst for promoting the glycerol hydrochlorination, surprisingly we have discovered a new class of very active and selective catalysts never proposed in the past constituted by acyl chlorides.

### Summary of the invention

The object of this invention is the process for the production of chlorohydrins starting from glycerol and gaseous hydrochloric acid using acyl chloride as catalysts for promoting the reaction. The catalysts of this invention have the following general formula RCOC1 and the following structure: 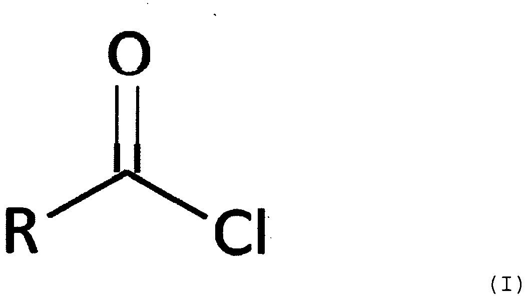 Where R is an alkyl chain with from 2 to 10 carbon atoms having a structure linear, branched or cyclic, eventually containing one or more R' groups, if more equal or different. R' groups are chosen between: linear alifatic groups, branched or cyclic with C₁-C₁₀. carbon atoms or aromatic (C₁-C₁₀), another or more than one acylic group.
By comparing the behavior of the acyl chlorides with the corresponding carboxylic acids it is possible to observe, in the same operative conditions, much greater reaction rates and final yields in α,γ-dichlorohydrin. By using acyl chloride the reaction time is strongly reduced as it will be seen in the examples reported later. This kinetic advantage becomes more and more evident by increasing the partial pressure of HCl. At last, it has been observed that inorganic salts, in particular inorganic chloride like sodium or potassium chloride have a further positive effect on the activity of the acyl chloride. This is very important for industrial purpose, because, allows the use of the crude glycerol coming from the biodiesel plants without any expensive purification step. It is known, in fact, that in biodiesel production NaOH or KOH are used as catalysts and at the end of the reaction the catalyst remains dissolved in the polar phase containing a mixture of methanol and glycerol. Then the catalyst is neutralized with mineral acids forming a salt that after the distillation of methanol remains dissolved in glycerol as impurity. We have discovered that this impurity if is a chloride has a positive effect on the reaction rate.
Main scope of this invention is to improve notably the efficiency and economy of the production of the α,γ-dichlorohydrin thanks to the employment of a new class of catalysts constituted by molecole containing one or more acylic functional groups.
Another scope of this invention is that related to the possibility of using crude glycerol coming from the biodiesel production limiting the post treatments to: neutralization with mineral acids, preferably HCl, of the base used as catalyst in biodiesel production and elimination of the residual methanol and of the eventual moisture.
Another scope of this invention is that of furnishing a process that allows to convert almost completely the glycerol.
A further scope of this invention is to furnish a process occurring in conditions that are thermodinamically favoured with yields and rates augmented with respect to the other processes.
Another additional scope of this invention is that to furnish a process that avoids the loss of catalyst during the reaction remaining the concentration of the catalyst high and constant in the reaction mixture. These and other scopes, that will better clarified in the following description of the invention, are accomplished by a process for the production of chlorohydrins, in particular α,γ-dichlorohydrin, including the reaction between glycerol and hydrochloric acid in the presence of a catalyst containing one or more acyl functional group.
Other objects of the invention will result evident from the following description reporting the operative detail of the invention.

### Brief description of the figures

The description of the invention will be better followed by observing the enclosed figures that represents not restrictive examples of a preferred form of realization.
**Figure 1****.** Scheme of the plant used to perform the runs reported in the examples, with: 1 - Reactor in Hastelloy, volume 300 mL; 2 - HCl cylinder; 3 - Valve for withdrawing the samples to be analyzed; 4 - Magnedrive stirrer; 5 -Temperature indicator; 6 - Pressure indicator; 7 - System per suppressing the excess of HCl (two Drechsel bottles in serie containing a solution of NaOH).
**Figure 2****.** Instantaneous HCl consumption in NL/h and evolution with time of reaction temperature in the presence of acetic acid as catalyst.
**Figure 3****.** Reaction products distribution for the reaction performed in the presence of acetic acid as catalyst.
**Figure 4****.** Instantaneous HCl consumption in NL/h and evolution with time of reaction temperature in the presence of acetyl chloride as catalyst.
**Figure 5****.** Reaction products distribution for the reaction performed in the presence of acetyl chloride as catalyst.
**Figure 6****.** Instantaneous HCl consumption in NL/h and evolution with time of reaction temperature in the presence of acetyl chloride as catalyst and sodium chloride as impurity.
**Figure 7****.** Reaction products distribution for the reaction performed in the presence of acetyl chloride as catalyst and sodium chloride as impurity.
**Figure 8****.** Instantaneous HCl consumption in NL/h and evolution with time of reaction temperature in the presence of acetyl chloride as catalyst by operating at higher pressure of HCl (10 bars).
**Figure 9****.** Reaction products distribution for the reaction performed in the presence of acetyl chloride as catalyst by operating at higher pressure of HCl (10 bars).
**Figure 10****.** Instantaneous HCl consumption in NL/h and evolution with time of reaction temperature in the presence of 2% by mole of acetyl chloride.
**Figure 11****.** Reaction products distribution for the reaction performed in the presence 2% by mole of acetyl chloride.
**Figure 12****.** Instantaneous HCl consumption in NL/h and evolution with time of reaction temperature in the presence of 4% by mole of acetyl chloride.
**Figure 13****.** Reaction products distribution for the reaction performed in the presence of 4% by mole of acetyl chloride.
**Figure 14****.** Instantaneous HCl consumption in NL/h and evolution with time of reaction temperature in the presence of propionic acid.
**Figure 15****.** Reaction products distribution for the reaction performed in the presence of propionic acid.
**Figure 16****.** Instantaneous HCl consumption in NL/h and evolution with time of reaction temperature in the presence of propanoyl chloride.
**Figure 17****.** Reaction products distribution for the reaction performed in the presence of propanoyl chloride as catalyst.
**Figure 18****.** Instantaneous HCl consumption in NL/h and evolution with time of reaction temperature in the presence of adipic acid.
**Figure 19****.** Reaction products distribution for the reaction performed in the presence of adipic acid.
**Figure 20****.** Instantaneous HCl consumption in NL/h and evolution with time of reaction temperature in the presence of esandioil chloride as catalyst.
**Figure 21****.** Reaction products distribution for the reaction performed in the presence of esandioil di-chloride as catalyst.
**Figure 22****.** Instantaneous HCl consumption in NL/h and evolution with time of reaction temperature in the presence of succinic acid as catalyst.
**Figure 23****.** Reaction products distribution for the reaction performed in the presence of succinic acid as catalyst.
**Figure 24****.** Instantaneous HCl consumption in NL/h and evolution with time of reaction temperature in the presence of butandioil di-chloride.
**Figure 25****.** Reaction products distribution for the reaction performed in the presence of butandioil di-chloride as catalyst.
**Figure 26****.** Instantaneous HCl consumption in NL/h and evolution with time of reaction temperature in the presence of malonic acid as catalyst.
**Figure 27****.** Reaction products distribution for the reaction performed in the presence of malonic acid as catalyst.
**Figure 28****.** Instantaneous HCl consumption in NL/h and evolution with time of reaction temperature in the presence of propandioil di-chloride as catalyst.
**Figure 29****.** Reaction products distribution for the reaction performed in the presence of propandioil di-chloride as catalyst.
**Figure 30****.** Instantaneous HCl consumption in NL/h and evolution with time of reaction temperature in the presence of phenylacetyl chloride as catalyst
**Figure 31****.** Reaction products distribution for the reaction performed in the presence of phenylacetyl chloride as catalyst.

### Detailed description of the invention

The process, according to this invention, consists in contacting glycerol with gaseous HCl in the presence of acyl chlorides of general formula (I) that are preliminarly dissolved in glycerol. Acyl chlorides pure or in mixtures act as catalyst of the hydrochlorination reaction. The catalyst can be used in a concentration range 1-20% (moles of acyl chloride/mole of glycerol %), most preferably in the range 5-10%. Hydrochloric acid is preferably used in gaseous anhydrous form. The use of gaseous HCl avoid the introduction of water in the reaction system. Water has a negative effect on both the reaction rate and the equilibrium.

The feeding of HCl to the reactor is regulated by keeping constant the pressure in a range of 0.5-60 bars, more preferably in a range of 5-20 bars. A high gas-liquid interface area is necessary to avoid mass transfer limitation. The high gas-liquid interface area can be achieved with different methods: high stirring rate, use of spray injector, use of Venturi jet tube, use of opportune static mixers, the use of tray tower etc.

However, the use of aqueous hydrochloric acid is not excluded from this invention.

The process of this invention can be conducted by using a semibatch reactor with a continuous feed of HCl to the reactor for substituting the consumed reagent. However, the process can also be conducted in a continuous reactor by feeding a continuous flow of both glycerol and HCl in continuous, stripping continuously α,γ-dichlorohydrin produced and recycling the catalyst and the unreacted HCl.

The reaction temperature can be chosen in the range 60-250°C most preferably in the range 100-175°C. By considering the relevant activity shown by the catalysts of this invention it is also possible to operate at high temperature (150-175°C) and elevated pressure (20-30 bars) in opportune continuous microreactors equipped with efficient static mixer with the scope of increasing very much the mass transfer rate and intensify the process with a strong reduction of the reactor volume.

Can be employed as catalyst the acyl chlorides having the previously reported general formula. Examples of acyl chlorides not reductive that can be used in this invention are: acetyl chloride, propionyl chloride, esandioil di-chloride, propandioil di-chloride, butandioil di-chloride, phenyl acetyl chloride.

As already mentioned the use of gaseous HCl pure or diluted with an inert gas is a preferred condition for this process in respect to the use of aqueous HCl, because, the efficiency and economy of the process are greatly improved. The use of gaseous HCl avoid the introduction of water in the preliminary phase of the reaction. Water is known has a detrimental effect on both the reaction rate and the yield.

In the examples that will be reported later gaseous HCl has been fed in a well stirred reactor. Some samples were withdrawn, at different reaction times, for the analysis of the products distribution determing the evolution with time of both reagent and products. The HCl consumption, at different reaction times has been determined with a flowmeter.

In the examples reported below that have an illustrative scope and not reductive of this invention are described in details different runs performed by reacting glycerol with gaseous HCl in the presence of different catalysts and with different modalities to produce chlorohydrins, in particular α,γ-dichlorohydrin. Some examples compare the behavior as catalyst of an acyl chloride with the behavior of the corresponding carboxylic acid with the scope to show the superiority of the acyl chloride in both activity and selectivity.

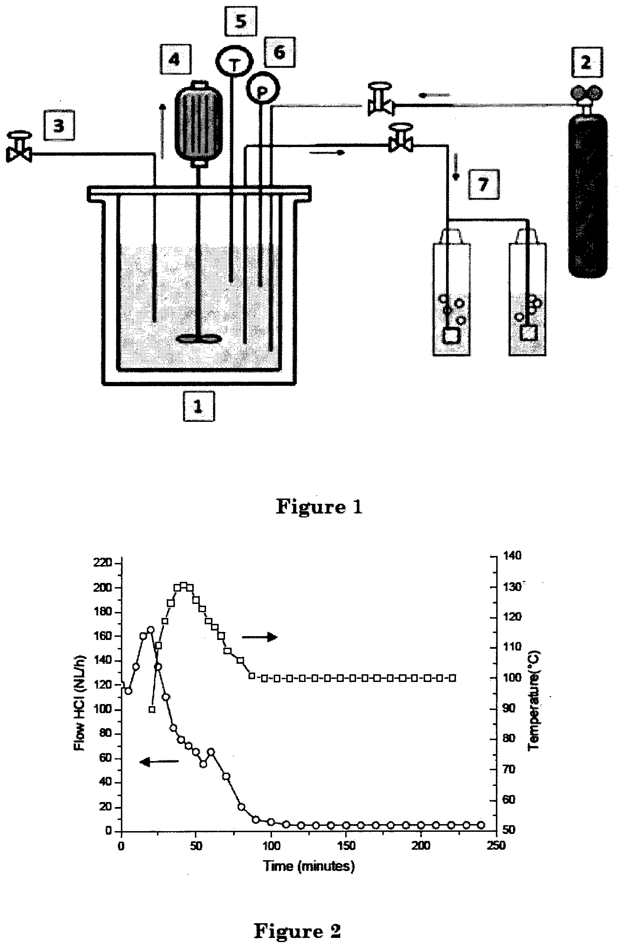

In Fig. 1, the scheme of the plant employed for making the runs of the examples, is reported. The reactor (1) is a Parr autoclave of 300 cm³ of volume, heated externally with a thermoregulated oven for obtaining a pre-fixed temperature. A weighed amount of glycerol is introduced in the reactor and mixed with a desired amount of catalyst (for example 8% mole of catalyst/mole of glycerol %). A magnetic driven stirrer (4) is normally kept at 1000 rpm. Then, the reactor is heated until reaching the reaction temperature. At this point HCl is fed from the cylinder (2) to the reactor keeping constant the pressure with the valveon the line.

Small amounts of the reaction mixture are withdrawn at different reaction times and analyzed to determine the composition. The obtained results are described in the following examples in which a detailed comparison is shown between the performances respectively obtained, in the same operative conditions, by using as catalysts acyl chlorides or the corresponding carboxylic acid.

In Figure 2, for example, is reported the instantaneous consumption of HCl, as a consequence of the reaction, at different reaction time by using acetic acid as catalyst. In the same figure is also reported the evolution with time of the reaction temperature.

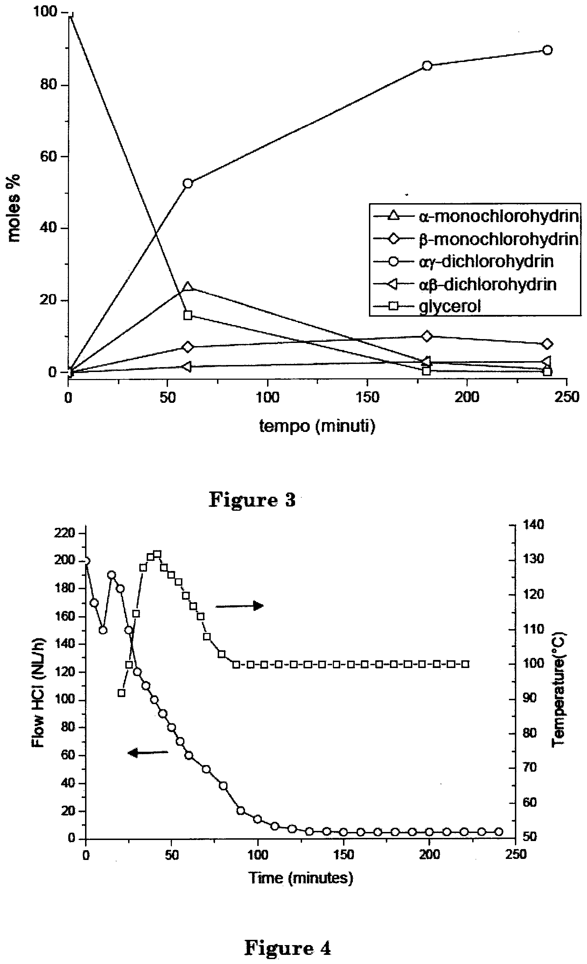

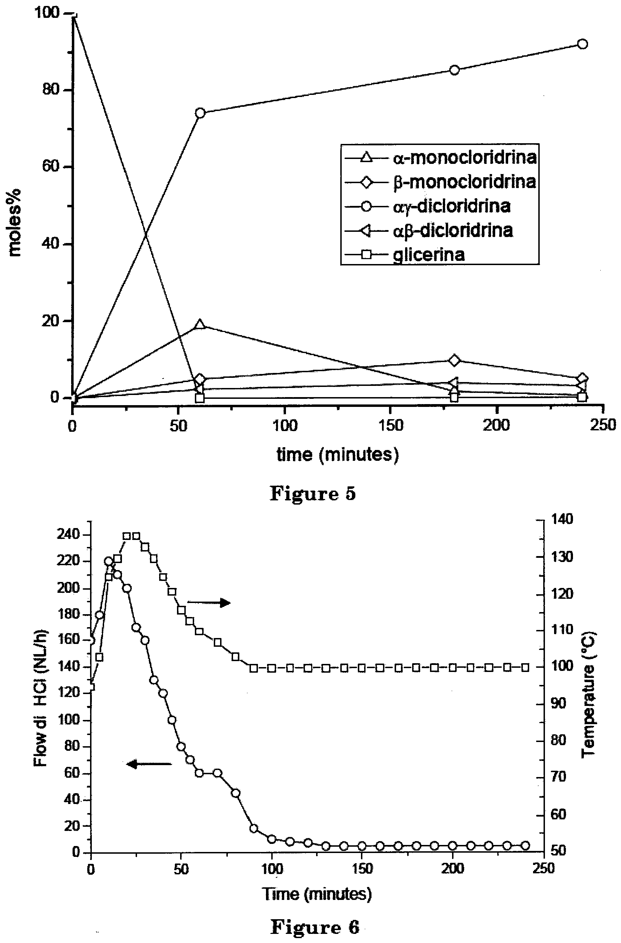

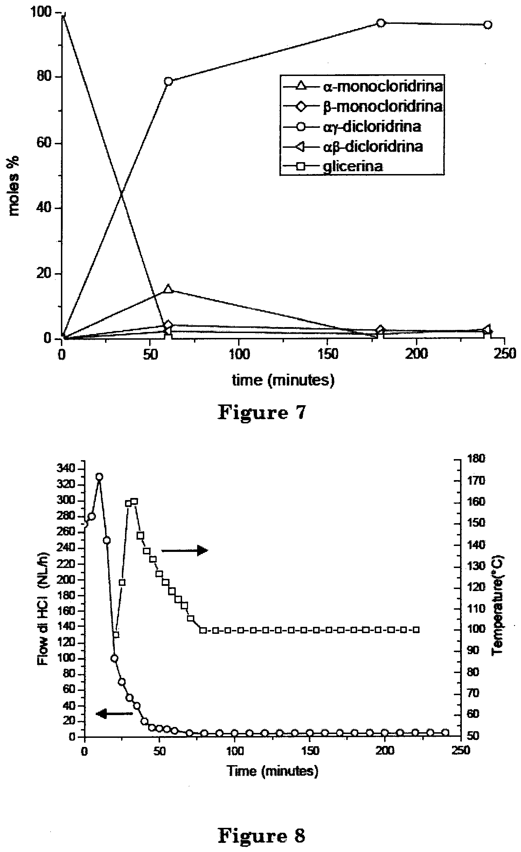

In Figure 3 the evolution with time of the product distribution, always using acetic acid as catalyst, is reported. These figures and related Tables (Table 1 and 2), must be compared with Figure Figure 4 and 5 and Tables 3 and 4 in which the same type of data, collected in the same conditions, but using acetyl chloride as catalyst, are reported. These comparison runs constitute the content of the Example 1 and as it can be seen, by using acetyl chloride as catalyst, the reagent glycerol completely disappears in a much shorter time and the final yield in α,γ-dichlorohydrin is higher than in the presence of acetic acid as catalyst. In all the successive examples, two figures similar to the ones shown in the Example 1 are reported, that is, a plot representing the instantaneous consumption of HCl during the time and a plot in which the evolution with time of the product distribution is reported. Moreover, two tables are reported for each run one containing the operative conditions adopted in the run and another reporting the composition of the reaction mixture at different reaction time. In the Example 2, characterized by the Figures 6 and 7 and from the Tables 5 and 6, is shown the positive effect of the presence of NaCl in the reacting mixture when acetyl chloride is used as catalyst. As it can be seen, in the presence of NaCl both the reaction rate and the selectivity are significantly increased. This effect, illustrated in the Example 2, open the possibility of using crude not-refined glycerol, coming from the plants of biodiesel, as feedstock for this process. This represents an evident economic advantage of this invention.

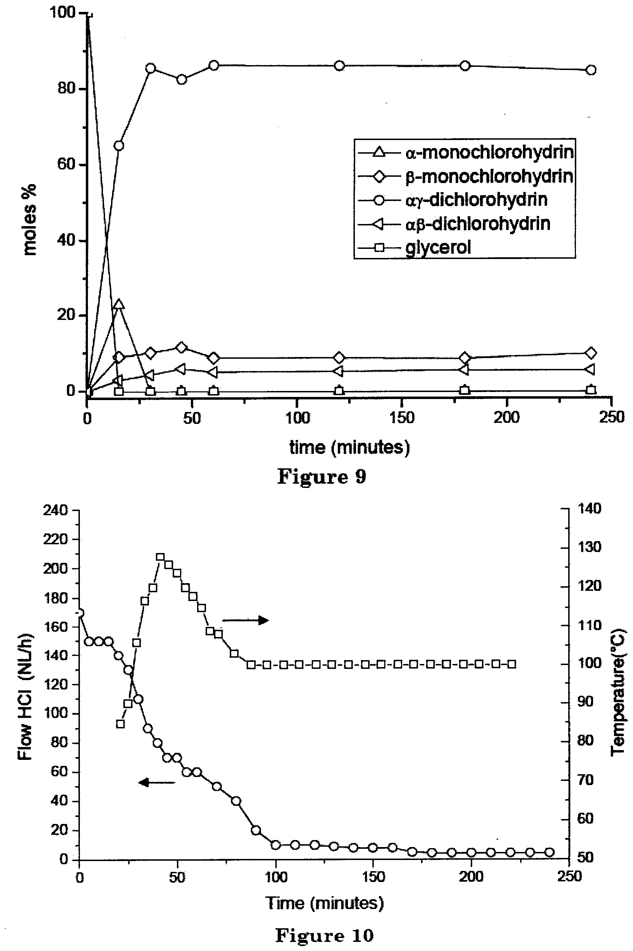

In the Example 3 (Figure 8 and 9 and corresponding Tables 7 and 8) the influence of the pressure of HCl on the reaction rate, when an acyl chloride is used as catalyst, is emphasized. By doubling approximately the pressure of HCl from 4.5 to 10 bars, always in the presence of acetyl chloride as catalyst, all the glycerol reagent is converted in less than 15 minutes, while, the maximum yield of α,γ-dichlorohydrin is reached in about 30 minutes. On the contrary, in the run performed at 4.5 bars using acetyl chloride as catalyst, the complete conversion of glycerol occurs in about 60 minutes. A run performed in the same conditions in the presence of acetic acid as catalyst requires more than 180 minutes for a total conversion of glycerol. In conclusion, the acyl chloride catalysts become more and more active at high pressure of HCl.

In the Example 4, characterized by the Figures 10-13 with related Tables 9-12 there is a comparison of the performance obtained, in the same operative conditions, by using different concentrations of acetyl chloride catalyst. The concentrations used in the Examples are respectively 2 and 4 % by mole and must be compared with the run reported in the Example 1b performed in the presence of 8% by mole of acetyl chloride.

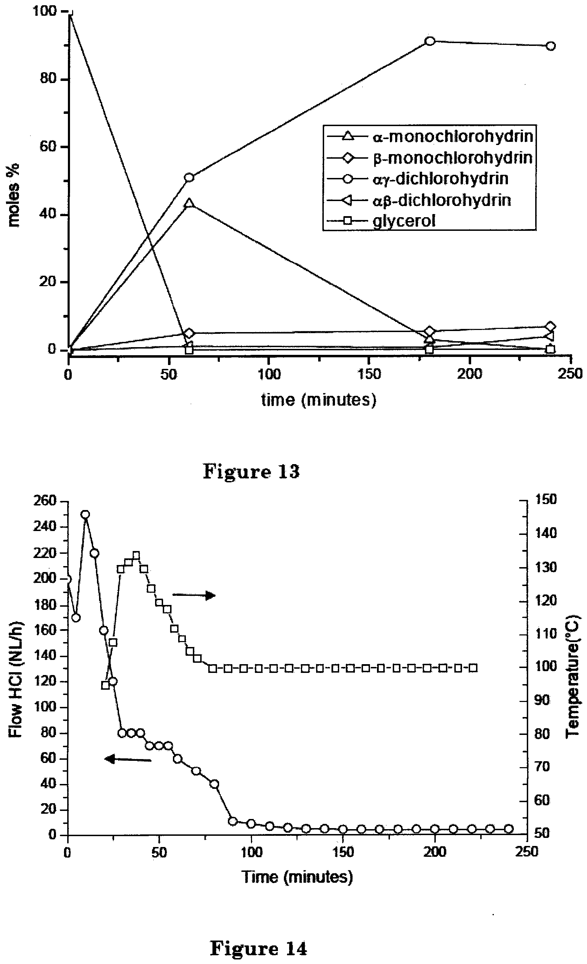

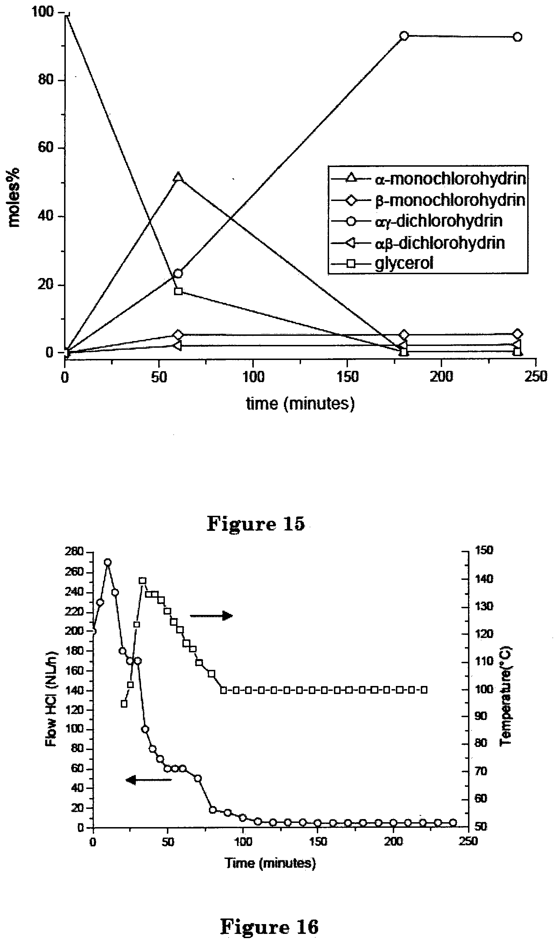

In the Example 5, characterized by the Figures 14-15 with related Tables 13-14 and by Figures 16-17 and related Table 15-16 there is a comparison of the performance obtained in the same operative conditions by respectively propanoic acid and propanoyl chloride. This comparison confirms the superiority as catalysts of the acyl chlorides with respect to the corresponding carboxylic acids. Glycerol, in the presence of propanoyl chloride is completely converted in less than 30 minutes, while, propanoic acid requires about 3 hours. Also the final yield of α,γ-dichlorohydrin is higher, 93.6% for the propanoyl chloride, 92.5% for the propanoic acid.

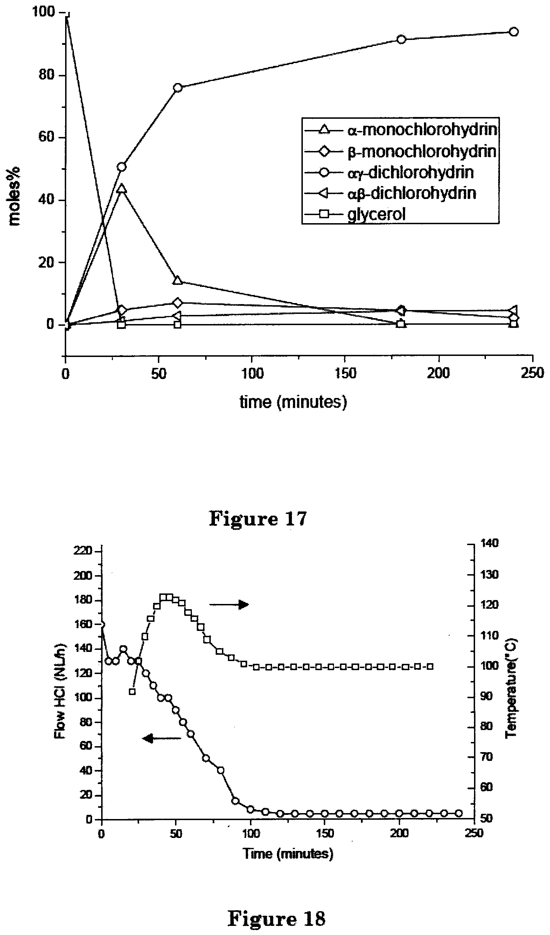

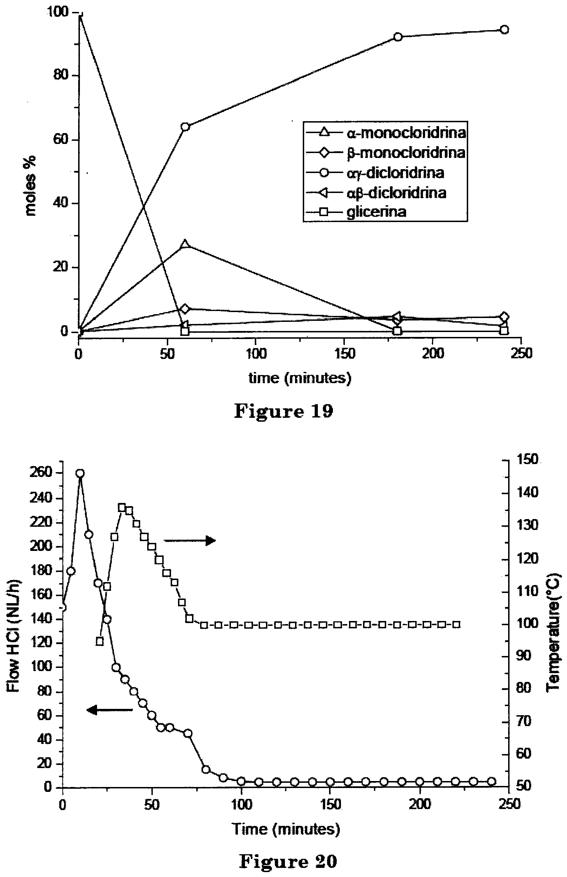

In the Example 6 there is the comparison of the performances obtained in the presence of respectively adipic acid and esandioil di-chloride. The comparison is illustrated by the plots of Figures 18-19 with related Tables 17-18 and Figures 20-21 with reale Tables 19-20. Also in this case the result of the comparison is dramatic. By using esandioil di-chloride as catalyst a yield of 92.8% of α,γ-dichlorohydrin is obtained in about 60 minutes, while, by using adipic acid 92% of yield is obtained in 180 minutes. It is confirmed that acyl chlorides are more active catalysts than carboxylic acids. In particular, the presence of two acyl chloride group in the same molecule enhances the catalytic effect.

In the Example 7 the performances as catalysts of respectively the succinic acid and the butandioil di-chloride are compared through the observation of the

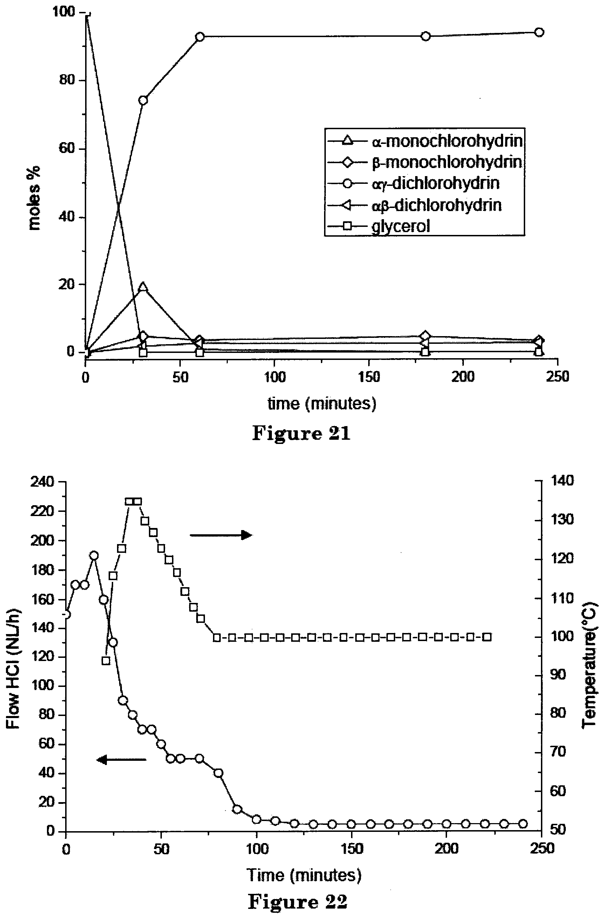

Figures 22-25 and related Tables 21-24. Also in this case it is possible to observe a superiority of the catalyst butandioil di-chloride with respect to the succinic acid. Glycerol concentration is zeroed in less than 60 minutes in the presence of butandioil di-chloride, while, 180 minutes are necessary for obtaining the same performance using succinic acid as catalyst. The maximum yield in α,γ-dichlorohydrin is obtained in 180 minutes (92.7%) in the presence of butandioil di-chloride and in 240 minutes (91.5) in the presence of succinic acid.

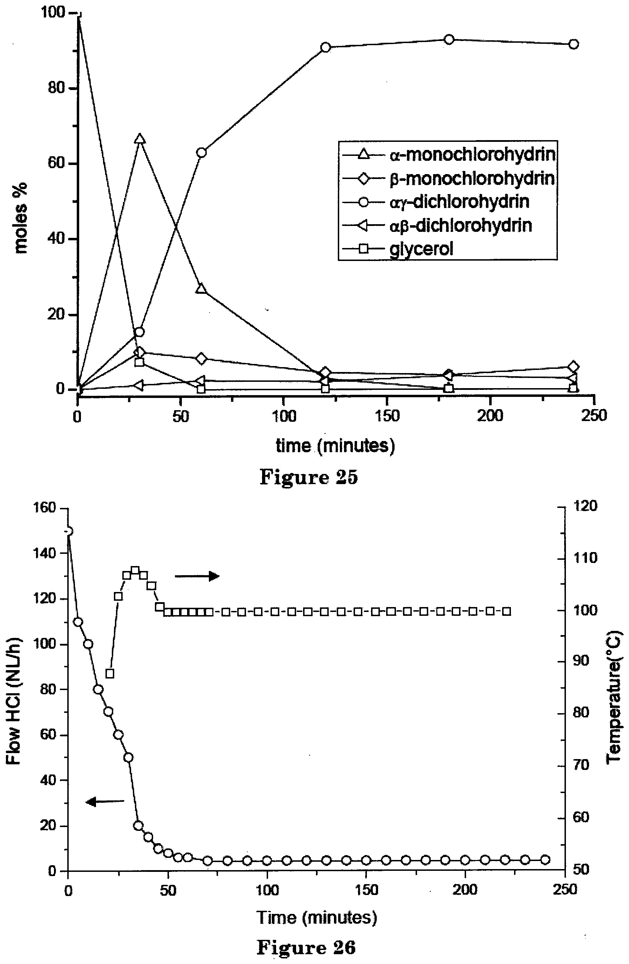

In the Example 8 is reported the comparison between the performances respectively obtained by using malonic acid and propandioil di-chloride through the observation of the Figures 26-29 and related Tables 25-28. Although propandioil di-chloride is less efficient than the other previously described acyl chloride it is more active than malonic acid further confirming the superiority of the acyl chlorides with respect to the corresponding carboxylic acids.

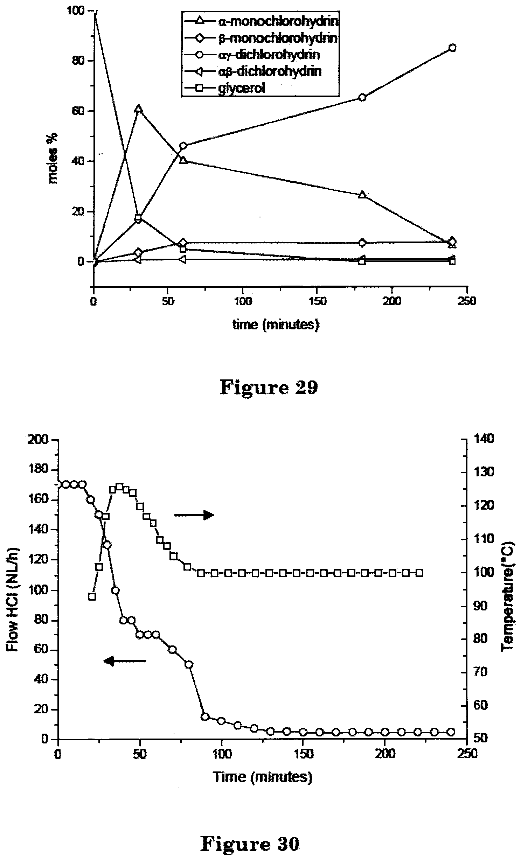

In the Example 9 are reported the results obtained by using phenylacetyl chloride as catalyst. The obtained results are reported in Figures 30-31 and the corresponding Tables 29-30. Also this acyl chloride has given very good performances converting all the glycerol in less than 60 minutes and giving the maximum yield of α,γ-dichlorohydrin in less than 180 minutes.

From all these results it is possible to conclude that acyl chlorides are much more active as catalysts than the corresponding carboxylic acids and give place also to higher selectivity in a shorter time.

It has been highlighted a synergic effect of NaCl with respect to the catalytic action of an acyl chloride catalyst opening the possibility to use crude glycerol from biodiesel plants as raw material instead of refined glycerol.

It has been observed that the partial pressure of HCl has a dramatic effect on the reaction rate when acyl chloride are used as catalysts.

Other factors that positively affect the reaction rate are: the catalyst concentration and the temperature.

In the next section, all the mentioned Examples will be described in detail. These examples have the scope to illustrate the invention and are not restrictive of the more general aspects of this invention.

### Examples

### Example 1- Reaction in the presence of acetyl chloride and comparison with acetic acid.

### a) Run in the presence of acetic acid (CAS-64-19-7)

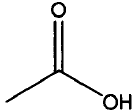

In Tab. 1 are reported the experimental conditions adopted for the run performed in the presence of acetic acid as catalyst. In Fig. 2 is reported the instantaneous consumption of HCl, in NL/h, monitored during the run and the evolution with time of the temperature. In Tab. 2 is reported the products distribution, determined at different reaction time. The same data are plotted in Figure 3.

**Table 1. Operative conditions and initial data.**

| | | |
|---|---|---|
| **Glycerol** | - | 150.0 g |
| **Amount of catalyst** | 8 % mols | 7.82 g |
| **Reaction time** | 240 minutes | - |
| **Fixed temperature** | 100 °C | - |
| **Pressure** | 4.5 bar | - |
| **Stirring rate** | 30.6 Hz | 1000 rpm |

**Tabella 2. Products distribution obtained by gas-chromatographic analysis.**

| **Time (minut es)** | **α-chlorohy drin** | **β-chlorohy drin** | **α,γ-dichlorohy drin** | **αβ-di chlorohydri n** | **Glyce rol** |
|---|---|---|---|---|---|
| 0 | 0 | 0 | 0 | 0 | 100 |
| 60 | 23.59 | 6.99 | 52.58 | 1.59 | 15.82 |
| 180 | 2.65 | 9.86 | 85.11 | 2.85 | 0.26 |
| 240 | 0.7 | 7.66 | 89.37 | 2.85 | 0 |

### b) Run in the presence of acetyl chloride (CAS-75-36-5)

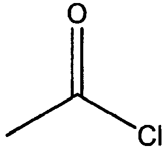

In Tab. 3 are reported the experimental conditions adopted for the run performed in the presence of acetyl chloride as catalyst. In Fig. 4 is reported the instantaneous consumption of HCl, in NL/h, monitored during the run and the evolution with time of the temperature. In Tab. 4 is reported the products distribution, determined at different reaction time. The same data are plotted in Fig.5. As it can be seen the use of acetyl chloride as catalyst determines a consistent improvement of both the activity and selectivity toward α,γ-dichlorohydrin.

**Table 3. Operative conditions and initial data.**

| | | |
|---|---|---|
| **Glycerol** | - | 150.0 g |
| **Amount of catalyst** | 8 % mols | 10.20 g |
| **Reaction time** | 240 minutes | - |
| **Fixed temperature** | 100 °C | - |
| **Pressure** | 4.5 bar | - |
| **Stirring rate** | 30.6 Hz | 1000 rpm |

**Table 4. Products distribution obtained by gas-chromatographic analysis.**

| **Time (minut es)** | **α-chlorohyd rin** | **β-chlorohyd rin** | **α,γ-dichlorohy drin** | **αβ-di chlorohyd rin** | **Glycer ol** |
|---|---|---|---|---|---|
| 0 | 0 | 0 | 0 | 0 | 100 |
| 60 | 18.8 | 4.9 | 74 | 2.3 | 0 |
| 180 | 1.6 | 9.6 | 85 | 3.8 | 0 |
| 240 | 0.5 | 4.8 | 91.7 | 3 | 0 |

### Example 2 - Influence of NaCl on the catalytic action of acetyl chloride.

In Tab. 5 are reported the experimental conditions adopted for the run performed in the presence of acetyl chloride as catalyst with the addition of NaCl. In Fig. 6 is reported the instantaneous consumption of HCl, in NL/h, monitored during the run and the evolution with time of the temperature. In Tab. 6, is reported the products distribution, determined at different reaction time. The same data are plotted in Figure 7. As it can be seen from the data of Tab. 6 and Figure 7 the presence of NaCl has a positive effect on the reaction. This open the possibility of using the unrefined glycerol, coming from the biodiesel plants where is obtained as by-product, as raw material for the process of this invention.

**Table 5 Operative conditions and initial data.**

| | | |
|---|---|---|
| **Glycerol** | - | 150.0 g |
| **Amount of catalyst** | 8 % mol | 10.20 g |
| **NaCl** | 8% wt | 12.0 g |
| **Reaction time** | 240 minutes | - |
| **Fixed temperature** | 100 °C | - |
| **Pressure** | 4.5 bar | - |
| **Stirring rate** | 30.6 Hz | 1000 rpm |

**Table 6. Products distribution obtained by gas-chromatographic analysis.**

| **Time (minut es)** | **α-chlorohyd rin** | **β-chlorohyd rin** | **α,γ-dichlorohy drin** | **αβ-di chlorohyd rin** | **Glycer ol** |
|---|---|---|---|---|---|
| 0 | 0 | 0 | 0 | 0 | 100 |
| 60 | 15 | 4.1 | 78.7 | 2.2 | 0 |
| 180 | 0 | 2.4 | 96.4 | 1.2 | 0 |
| 240 | 0 | 1.8 | 95.7 | 2.5 | 0 |

### Example 3 - Reaction in the presence of acetyl chloride at higher pressure of HCl.

This run show the dramatic influence of the HCl pressure on the reaction rate. In Tab. 7 are reported the experimental conditions adopted for the run performed in the presence of acetyl chloride at higher pressure. In Fig. 8 is reported the instantaneous consumption of HCl, in NL/h, monitored during the run and the evolution with time of the temperature. In Tab. 8, is reported the products distribution, determined at different reaction time. The same data are plotted in Figure 9. By comparing this run with the one reported in the Example 1, run 1b, it can be observed that the pressure of HCl has a very great influence on the reaction rate.

**Table 7 Operative conditions and initial data.**

| | | |
|---|---|---|
| **Glycerol** | - | 150.0 g |
| **Amount of catalyst** | 8 % mol | 10.20 g |
| **Reaction time** | 240 minuti | - |
| **Fixed temperature** | 100 °C | - |
| **Pressure** | 10 bar | - |
| **Stirring rate** | 30.6 Hz | 1000 rpm |

**Table 8. Products distribution obtained by gas-chromatographic analysis.**

| **Time (minut es)** | **α-chlorohy drin** | **β-chlorohyd rin** | **α,γ-dichlorohyd rin** | **αβ-di chlorohydr in** | **Glyce rol** |
|---|---|---|---|---|---|
| 0 | 0 | 0 | 0 | 0 | 100 |
| 15 | 22.9 | 9.1 | 65 | 3 | 0 |
| 30 | 0 | 10.2 | 85.4 | 4.4 | 0 |
| 45 | 0 | 11.6 | 82.4 | 6 | 0 |
| 60 | 0 | 8.8 | 86.1 | 5.1 | 0 |
| 120 | 0 | 8.8 | 85.9 | 5.3 | 0 |
| 180 | 0 | 8.6 | 85.8 | 5.6 | 0 |
| 240 | 0 | 9.8 | 84.6 | 5.6 | 0 |

### Example 4 - Reaction in the presence of acetyl chloride, the effect of the catalyst concentration.

In this examples are reported two runs performed with a acetyl chloride molar concentration of respectively 2 and 4% that must be compared with the run performed by using 8% of acetyl chloride, reported in the Example 1b.

### a) Run performed with 2% of acetyl chloride

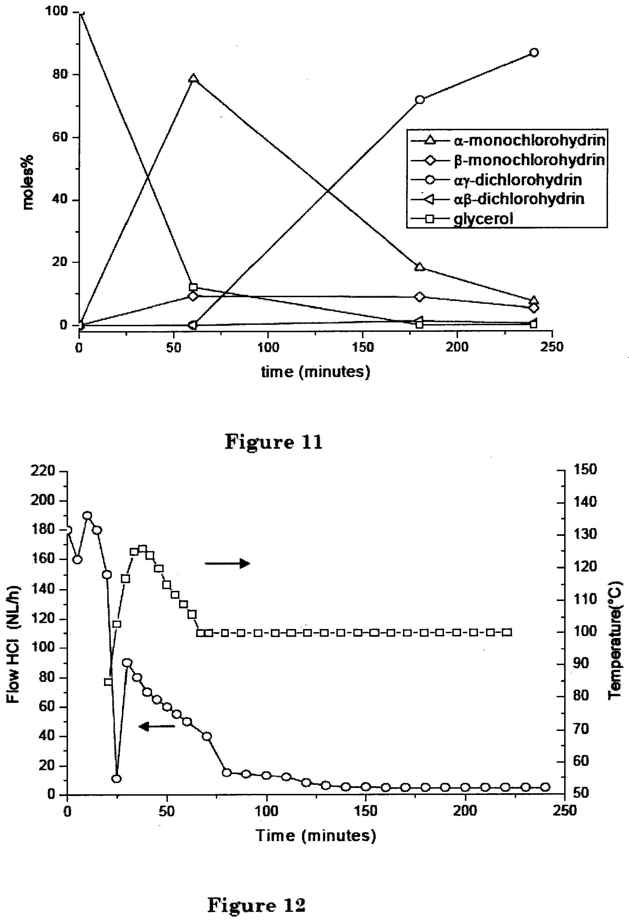

In Tab. 9 are reported the experimental conditions adopted for the run performed in the presence of acetyl chloride with concentration reduced at 2%. In Fig. 10 is reported the instantaneous consumption of HCl, in NL/h, monitored during the run and the evolution with time of the temperature. In Tab. 10, is reported the products distribution, determined at different reaction time. The same data are plotted in Figure 11.

**Table 9 Operative conditions and initial data.**

| | | |
|---|---|---|
| **Glycerol** | - | 150.0 g |
| **Amount of catalyst** | 2 % mol | 2.55 g |
| **Reaction time** | 240 minuti | - |
| **Fixed temperature** | 100 °C | - |
| **Pressure** | 4.5 bar | - |
| **Stirring rate** | 30.6 Hz | 1000 rpm |

**Table 10. Products distribution obtained by gas-chromatographic analysis.**

| **Time (minutes)** | **α-chlorohydrin** | **β-chlorohydrin** | **α,γ-dichlorohydrin** | **αβ-di chlorohydrin** | **glycerol** |
|---|---|---|---|---|---|
| 0 | 0 | 0 | 0 | 0 | 100 |
| 60 | 78,7 | 9,2 | 0 | 0 | 12,1 |
| 180 | 18,1 | 8,8 | 71,8 | 1,3 | 0 |
| 240 | 7,4 | 5,3 | 86,8 | 0,5 | 0 |

### b) Run performed with 2% of acetyl chloride

In Tab. 11 are reported the experimental conditions adopted for the run performed in the presence of acetyl chloride with concentration reduced at 4%. In Fig. 12 is reported the instantaneous consumption of HCl, in NL/h, monitored during the run and the evolution with time of the temperature. In Tab. 12, is reported the products distribution, determined at different reaction time. The same data are plotted in Figure 13.

**Table 11- Operative conditions and initial data.**

| | | |
|---|---|---|
| **Glycerol** | - | 150.0 g |
| **Amount of catalyst** | 2 % mol | 5.1 g |
| **Reaction time** | 240 minuti | - |
| **Fixed temperature** | 100 °C | - |
| **Pressure** | 4.5 bar | - |
| **Stirring rate** | 30.6 Hz | 1000 rpm |

**Table 12. Products distribution obtained by gas-chromatographic analysis.**

| **Time (minutes)** | **α-chlorohydrin** | **β-chlorohydrin** | **α,γ-dichlorohydrin** | **αβ-di chlorohydrin** | **glycerol** |
|---|---|---|---|---|---|
| 0 | 0 | 0 | 0 | 0 | 100 |
| 60 | 43,1 | 4,9 | 50,8 | 1,2 | 0 |
| 180 | 2,9 | 5,4 | 91 | 0,7 | 0 |
| 240 | 0 | 6,7 | 89,5 | 3,8 | 0 |

As it can be seen by comparing the data reported in this Example with the ones of the Example 1b, by reducing the catalyst concentration the reaction rate decreases but not very much, because in all cases glycerol reagent is completely converted in less than one hour but the concentration of the intermediate α-chlorohydrin after one hour is 18.8 % for the run performed with 8% of acetyl chloride, 43.1% for the run using 4% of catalyst and 78.7% for the run using 2% of catalyst. The time to reach the maximum yield is approximately the same for the runs performed at 4 or at 8% of catalyst (that is between 3-4 hours), while, is longer for the run performed with 2% of catalyst. This means that the optimal catalyst concentration is in the range 4-8% in mole of acyl chloride per mole of glycerol.

### Example 5 - Reaction in the presence of propionyl chloride and comparison with propionic acid.

### a) Run in the presence of propionic acid (CAS- 79-09-4)

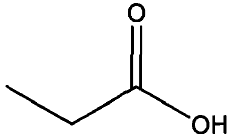

In Tab. 13 are reported the experimental conditions adopted for the run performed in the presence of propionic acid as catalyst. In Fig. 14 is reported the instantaneous consumption of HCl, in NL/h, monitored during the run and the evolution with time of the temperature. In Tab. 14 is reported the products distribution, determined at different reaction time. The same data are plotted in Figure 15.

**Table 13 - Operative conditions and initial data.**

| | | |
|---|---|---|
| **Glycerol** | - | 150.0 g |
| **Amount of catalyst** | 8 % mol | 9.60 g |
| **Reaction time** | 240 minuti | - |
| **Fixed temperature** | 100 °C | - |
| **Pressure** | 4.5 bar | - |
| **Stirring rate** | 30.6 Hz | 1000 rpm |

**Table 14 - Products distribution obtained by gas-chromatographic analysis.**

| **Time (minut es)** | **α-chlorohyd rin** | **β-chlorohyd rin** | **α,γ-dichlorohy drin** | **αβ-di chlorohyd rin** | **Glycer ol** |
|---|---|---|---|---|---|
| 0 | 0 | 0 | 0 | 0 | 100 |
| 60 | 51.3 | 5.3 | 23.2 | 2.1 | 18.1 |
| 180 | 0 | 5.1 | 92.9 | 2 | 0 |
| 240 | 0 | 5.3 | 92.5 | 2.2 | 0 |

### b) Run in the presence of propanoyl chloride (CAS- 79-03-8)

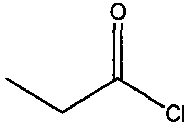

In Tab. 15 are reported the experimental conditions adopted for the run performed in the presence of propanoyl chloride as catalyst. In Fig. 16 is reported the instantaneous consumption of HCl, in NL/h, monitored during the run and the evolution with time of the temperature. In Tab. 16 is reported the products distribution, determined at different reaction time. The same data are plotted in Figure 17.

As it can be seen the use of propanoyl chloride as catalyst determines a consistent improvement of both the activity and selectivity toward α,γ-dichlorohydrin.

**Table 15. Operative conditions and initial data.**

| | | |
|---|---|---|
| **Glycerol** | - | 150.0 g |
| **Amount of** | 8 % mol | 12.0 g |
| **catalyst** | | |
| **Reaction time** | 240 minutes | - |
| **Fixed temperature** | 100 °C | - |
| **Pressare** | 4.5 bar | - |
| **Stirring rate** | 30.6 Hz | 1000 rpm |

**Table 16. Products distribution obtained by gas-chromatographic analysis.**

| **Time (minute s)** | **α-chlorohyd rin** | **β-chlorohyd rin** | **α,γ-dichlorohyd rin** | **αβ-di chlorohyd rin** | **Glycer ol** |
|---|---|---|---|---|---|
| 0 | 0 | 0 | 0 | 0 | 100 |
| 30 | 43.4 | 4.8 | 50.6 | 1.2 | 0 |
| 60 | 14.1 | 7.1 | 75.9 | 2.9 | 0 |
| 180 | 0 | 4.5 | 91.2 | 4.3 | 0 |
| 240 | 0 | 2 | 93.6 | 4.4 | 0 |

### Example 6 - Reaction in the presence of esandioil di-chloride and comparison with adipic acid.

### a) Run in the presence of adipic acid (CAS-124-4-9)

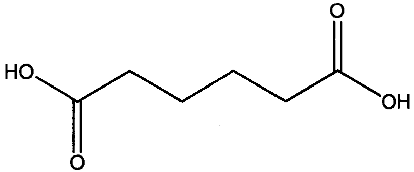

In Tab. 17 are reported the experimental conditions adopted for the run performed in the presence of adipic acid as catalyst. In Fig. 18 is reported the instantaneous consumption of HCl, in NL/h, monitored during the run and the evolution with time of the temperature. In Tab. 18 is reported the products distribution, determined at different reaction time. The same data are plotted in Figure 19.

**Table 17 Operative conditions and initial data.**

| | | |
|---|---|---|
| **Glycerol** | - | 150.0 g |
| **Amount of catalyst** | 8 % mol | 19.0 g |
| **Reaction time** | 240 minutes | - |
| **Fixed temperature** | 100 °C | - |
| **Pressure** | 4.5 bar | - |
| **Stirring rate** | 30.6 Hz | 1000 rpm |

**Table 18. Products distribution obtained by gas-chromatographic analysis.**

| **Time (minut es)** | **α-chlorohyd rin** | **β-chlorohyd rin** | **α,γ-dichlorohyd rin** | **αβ-di chlorohyd rin** | **Glycer ol** |
|---|---|---|---|---|---|
| 0 | 0 | 0 | 0 | 0 | 100 |
| 60 | 27 | 7 | 64 | 2 | 0 |
| 180 | 0 | 3.5 | 92 | 4.5 | 0 |
| 240 | 0 | 4.3 | 94.2 | 1.5 | 0 |

### b) Run with esandioil di-chloride (CAS- 111-50-2)

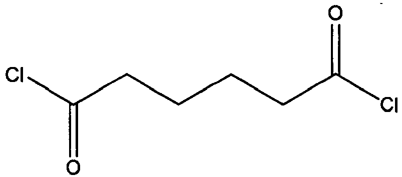

In Tab. 19 are reported the experimental conditions adopted for the run performed in the presence of esandioil di-chloride as catalyst. In Fig. 20 is reported the instantaneous consumption of HCl, in NL/h, monitored during the run and the evolution with time of the temperature.

In Tab. 20 is reported the products distribution, determined at different reaction time. The same data are plotted in Figure 21. Also in this case the superiority of the acyl chlorides as catalyst is confirmed.

**Table 19 Operative conditions and initial data.**

| | | |
|---|---|---|
| **Glycerol** | - | 150.0 g |
| **Amount of catalyst** | 8 % mol | 23.8 g |
| **Reaction time** | 240 minutes | - |
| **Fixed temperature** | 100 °C | - |
| **Pressure** | 4.5 bar | - |
| **Stirring rate** | 30.6 Hz | 1000 rpm |

**Table 20. Products distribution obtained by gas-chromatographic analysis.**

| **Time (minut es)** | **α-chlorohyd rin** | **β-chlorohyd rin** | **α,γ-dichlorohyd rin** | **αβ-di chlorohyd rin** | **Glycer ol** |
|---|---|---|---|---|---|
| **0** | **0** | **0** | **0** | **0** | **100** |
| **30** | **19.2** | **4.8** | **74.1** | **1.9** | **0** |
| **60** | **0.9** | **3.6** | **92.8** | **2.7** | **0** |

| | | | | | |
|---|---|---|---|---|---|
| **180** | **0** | **4.6** | **92.8** | **2.6** | **0** |
| **240** | **0** | **3.3** | **93.9** | **2.8** | **0** |

### Example 7 - Reaction in the presence of butandioil di-chloride and comparison with succinic acid.

### a) Run in the presence of Succinic acid (CAS-110-15-6)

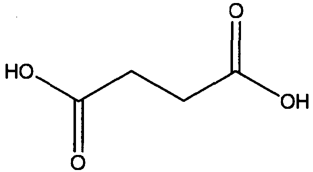

In Tab. 21 are reported the experimental conditions adopted for the run performed in the presence of esandioil di-chloride as catalyst. In Fig. 22 is reported the instantaneous consumption of HCl, in NL/h, monitored during the run and the evolution with time of the temperature.

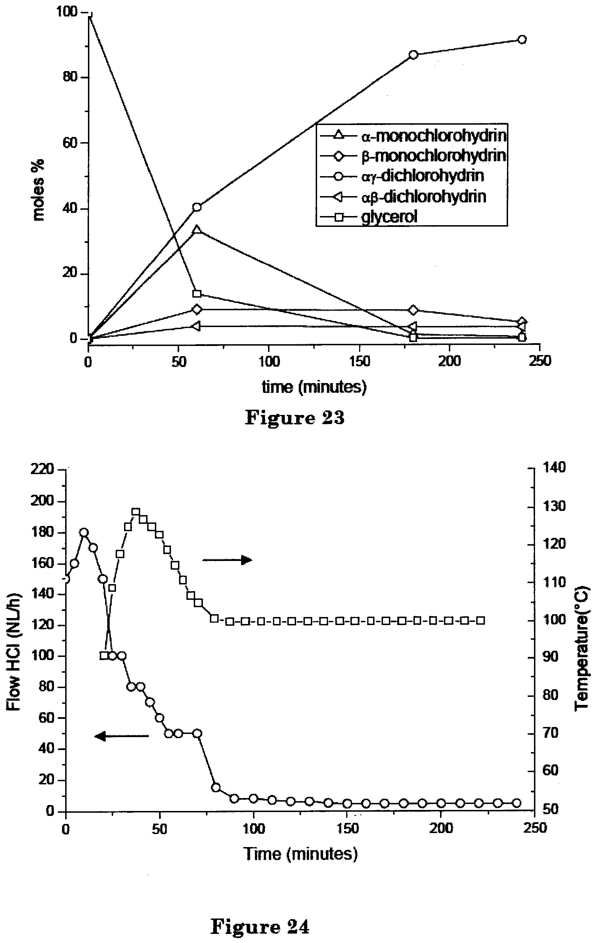

In Tab. 22 is reported the products distribution, determined at different reaction time. The same data are plotted in Figure 23.

**Table 21- Operative conditions and initial data.**

| | | |
|---|---|---|
| **Glycerol** | - | 150.0 g |
| **Amount of catalyst** | 8 % mol | 15.3 g |
| **Reaction time** | 240 minutes | - |
| **Fixed temperature** | 100 °C | - |
| **Pressure** | 4.5 bar | - |
| **Stirring rate** | 30.6 Hz | 1000 rpm |

**Table 22- Products distribution obtained by gas-chromatographic analysis.**

| **Time (minut es)** | **α-chlorohyd rin** | **β-chlorohyd rin** | **α,γ-dichlorohy rin** | **αβ-di chlorohyd rin** | **Glycer ol** |
|---|---|---|---|---|---|
| 0 | 0 | 0 | 0 | 0 | 100 |
| 60 | 33.2 | 9 | 40.3 | 3.8 | 13.7 |
| 180 | 1.1 | 8.5 | 86.9 | 3.5 | 0 |
| 240 | 0.3 | 4.8 | 91.5 | 3.4 | 0 |

### b) Run in the presence of butandioil di-chloride. (CAS 543-20-4)

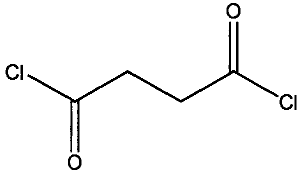

In Tab. 23 are reported the experimental conditions adopted for the run performed in the presence of butandioil di-chloride as catalyst. In Fig. 24 is reported the instantaneous consumption of HCl, in NL/h, monitored during the run and the evolution with time of the temperature.

In Tab. 24 is reported the products distribution, determined at different reaction time. The same data are plotted in Figure 25. Also in this case the superiority of the acyl chlorides as catalyst is confirmed.

**Table 23- Operative conditions and initial data.**

| | | |
|---|---|---|
| **Glycerol** | - | 150.0 g |
| **Amount of catalyst** | 8 % mol | 20.1 g |
| **Reaction time** | 240 minutes | - |
| **Fixed temperature** | 100 °C | - |
| **Pressure Stirring** | 4.5 bar | - |
| **rate** | 30.6 Hz | 1000 rpm |

**Table 24 - Products distribution obtained by gas-chromatographic analysis.**

| **Time (minut es)** | **α-chlorohyd rin** | **β-chlorohyd rin** | **α,γ-dichlorohyd rin** | **αβ-di chlorohyd rin** | **Glycer ol** |
|---|---|---|---|---|---|
| 0 | 0 | 0 | 0 | 0 | 100 |
| 30 | 66.4 | 9.9 | 15.3 | 1.1 | 7.3 |
| 60 | 26.6 | 8.2 | 62.9 | 2.3 | 0 |
| 120 | 2.8 | 4.5 | 90.7 | 2 | 0 |
| 180 | 0 | 3.8 | 92.7 | 3.5 | 0 |
| 240 | 0 | 5.8 | 91.4 | 2.8 | 0 |

### Example 8 - Reaction in the presence of propandioil di-chloride and comparison with malonic acid.

### a) Run in the presence of malonic acid (CAS-141-82-2)

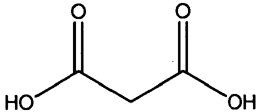

In Tab. 25 are reported the experimental conditions adopted for the run performed in the presence of malonic acid as catalyst. In Fig. 26 is reported the instantaneous consumption of HCl, in NL/h, monitored during the run and the evolution with time of the temperature.

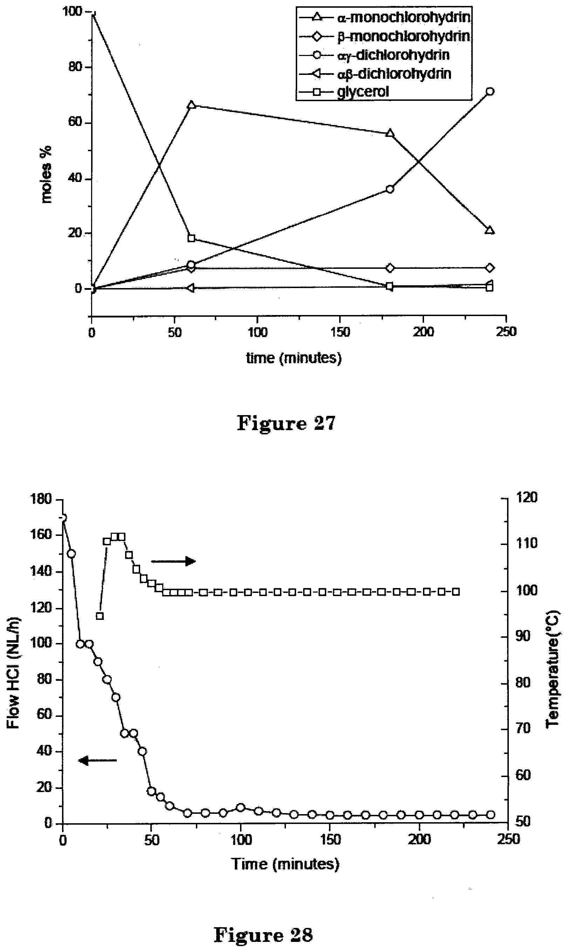

In Tab. 26 is reported the products distribution, determined at different reaction time. The same data are plotted in Figure 27.

**Table 25- Operative conditions and initial data.**

| | | |
|---|---|---|
| **Glycerol** | - | 150.0 g |
| **Amount of catalyst** | 8 % mol | 13.5 g |
| **Reaction time** | 240 minutes | - |
| **Fixed temperature** | 100 °C | - |
| **Pressure** | 4.5 bar | - |
| **Stirring rate** | 30.6 Hz | 1000 rpm |

**Table 26 - Products distribution obtained by gas-chromatographic analysis.**

| **Time (minut es)** | **α-chlorohyd rin** | **β-chlorohyd rin** | **α,γ-dichlorohyd rin** | **αβ-di chlorohyd rin** | **Glycer ol** |
|---|---|---|---|---|---|
| 0 | 0 | 0 | 0 | 0 | 100 |
| 60 | 66.3 | 7.4 | 8.6 | 0.2 | 18.1 |
| 180 | 55.8 | 7.2 | 35.9 | 0.5 | 0.6 |
| 240 | 20.6 | 7.2. | 71 | 1.2 | 0 |

### b) Run in the presence of propandioil di-chloride. (CAS -1663-67-8)

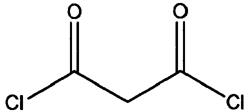

In Tab. 27 are reported the experimental conditions adopted for the run performed in the presence of propandioil di-chloride as catalyst. In Fig. 28 is reported the instantaneous consumption of HCl, in NL/h, monitored during the run and the evolution with time of the temperature. In Tab. 28 is reported the products distribution, determined at different reaction time. The same data are plotted in Figure 29. Also in this case the superiority of the acyl chlorides as catalyst is confirmed.

**Table 27- Operative conditions and initial data.**

| | | |
|---|---|---|
| **Glycerol** | - | 150.0 g |
| **Amount of catalyst** | 8 % mol | 18.3 g |
| **Reaction time** | 240 minutes | - |
| **Fixed temperature** | 100 °C | - |
| **Pressure** | 4.5 bar | - |
| **Stirring rate** | 30.6 Hz | 1000 rpm |

**Table 28 - Products distribution obtained by gas-chromatographic analysis.**

| **Time (minut es)** | **α-chlorohyd rin** | **β-chlorohyd rin** | **α,γ-dichlorohy drin** | **αβ-di chlorohyd rin** | **Glycer ol** |
|---|---|---|---|---|---|
| 0 | 0 | 0 | 0 | 0 | 100 |
| 30 | 60.8 | 3.7 | 16.8 | 0.8 | 17.9 |
| 60 | 40.2 | 7.6 | 46.2 | 1 | 5 |
| 180 | 26.4 | 7.4 | 65.4 | 0.8 | 0 |
| 240 | 6.3 | 7.8 | 85.1 | 0.8 | 0 |

### Example 9 - Run in the presence of phenylacetyl chloride (CAS-103-82-2)

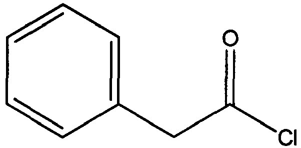

In Tab. 29 are reported the experimental conditions adopted for the run performed in the presence of phenylacetyl chloride as catalyst.

In Fig. 30 is reported the instantaneous consumption of HCl, in NL/h, monitored during the run and the evolution with time of the temperature.

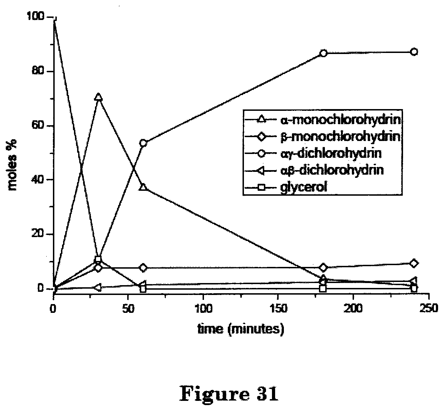

In Tab. 30 is reported the products distribution, determined at different reaction time. The same data are plotted in Figure 31.

**Table 29 Operative conditions and initial data.**

| | | |
|---|---|---|
| **Glycerol** | - | 150.0 g |
| **Amount of catalyst** | 8 % mol | 20.1 g |
| **Reaction time** | 240 minutes | - |
| **Fixed temperature** | 100 °C | - |
| **Pressure** | 4.5 bar | - |
| **Stirring rate** | 30.6 Hz | 1000 rpm |

**Table 30. Products distribution obtained by gas-chromatographic analysis.**

| **Time (minut es)** | **α-chlorohyd rin** | **β-chlorohyd rin** | **α,γ-dichlorohyd rin** | **αβ-di chlorohyd rin** | **Glycer ol** |
|---|---|---|---|---|---|
| 0 | 0 | 0 | 0 | I 0 | 100 |
| 30 | 70.3 | 7.8 | 10.5 | 0.5 | 10.9 |
| 60 | 37.1 | 7.8 | 53.5 | 1.6 | 0 |
| 180 | 3.5 | 7.7 | 86.5 | 2.3 | 0 |
| 240 | 1.1 | 9.2 | 87 | 2.7 | 0 |

### Literature

[1] Solvay Inc. Process for the production of alpha-epichlorhydrin. GB 799,567(1958).
[2] P. Bruin; Epoxy ethers, their polymers and derivatives. GB 814,695(1959).
[3] Z. Brojer, P. A. Penczec,; J. H. Sas; Process of manufacturing epoxide resins. GB 1,001,364, (1965)
[4] T. Kawabe, M. Kadoma, K. Murakami, H. Itoh; Process for continuous production of epichlorohydrin; US 4113746 (1978)
[5] S. Carrà, E. Santacesaria, and M. Morbidelli, Ind. Eng. Chem. Process Des. Dev., 18 (3), 424-427, (1979).
[6] S. Carrà, E. Santacesaria, and M. Morbidelli, Ind. Eng. Chem. Process Des. Dev., 18 (3), 428-433, (1979)
[7] Clarke, H. T.; Hartman, W. W. Epichlorohydrin. In Organic Syntheses, Coll.; (1941); Vol. 1, p 233.
[8] J. B. Conant, O. R. Quayle; Glycerol αγ-dichlorohydrin. In Organic Syntheses, Coll.; (1941); Vol. 1, p 292.
[9] J. B. Conant, O. R. Quayle; Glycerol α-monochlorohydrin. In Organic Syntheses, Coll.; (1941); Vol. 1, p 294.
[10] Fauconnier, Sanson, Bull. Soc. Chim., 48, (1887), pp. 236-240
[11] A. J. Hill, E. J. Fischer, A synthesis of beta-chloro-allyl chloride; J. Am. Chem. Soc., (1922) Vol. 44, pp. 2582-2595
[12] E. G. Britton, R. X. Heindel, Preparation of glycerol dichlorohydrin; US 2,144,612 (1939)
[13] E. C. Britton, H. R. Slagh; Glycerol dichlorohydrin; US 2,198,600 (1940)
[14] W. P. Thompson; Recovery of aliphatic chlorohydrins having from 3 to 5 carbon atoms from aqueous solution. GB 931,211 (1963).
[15] P. Krafft, P. Gilbeau, B. Gosselin, S. Claessens, (Solvay). Process for producing dichloropropanol from glycerol, the glycerol coming eventually from the conversion of animal fats in the manufacture of biodiesel. PCT Patent, WO 054167 A1, 2005.
[16] Siano, D.; Santacesaria, E.; Fiandra, V.; Tesser, R.; Di Nuzzi, G.; Di Serio, M.; Nastasi, M.. (Eurochem Engineering srl); Process for the production of α,γ-dichlorohydrin from glycerin and hydrochloric acid. WO 111810 A2, 2006 and US 2009/0062574
[17] D. Schreck, W. Kruper, R. D. Varjian, M. E. Jones, R. M. Campbell, K. Kearns, B. D. Hook, , J. R. Briggs, J. G. Hippler, (Dow Global Tech. Inc.). Conversion of multihydroxylated-aliphatic hydrocarbon or ester thereof to a chlorohydrin. PCT Patent, WO 2006/020234 and EP 1 771 403 B1 (2007)
[18] R. Tesser, M. Di Serio, R. Vitiello, V. Russo, E. Ranieri, E. Speranza, E. Santacesaria; Glycerol Chlorination in Gas_Liquid Semibatch Reactor: An Alternative Route for Chlorohydrins Production; Ind. Eng. Chem. Res. 51 (2012) 8768-8776.
[19] B. M. Bell, J. R. Briggs, R. M. Campbell, S. M. Chambers, P. D. Gaarenstroom, J. G. Hippler, B. D. Hook, K. Kearns, J. M. Kenney, W. J. Kruper, D. J. Schreck, C. N. Theriault, C. P. Wolfe, Glycerin as a Renewable Feedstock for Epichlorohydrin Production. The GTE Process, Clean 36 (2008) 657 - 661
[20] S. Cassarino, F. Simola; (CONSER SpA) Conversion of glycerine to dichlorohydrins and epichlorohydrin. WO 2009/066327
[21] E. Santacesaria, R. Tesser, M. Di Serio, L. Casale,; D. Verde, New Process for Producing Epichlorohydrin via Glycerol Chlorination; Ind. Eng. Chem. Res. 49 (2010) 964-970.
[22] R. Tesser, E. Santacesaria, M. Di Serio, G. Di Nuzzi, and V. Fiandra; Kinetics of Glycerol Chlorination with Hydrochloric Acid: A New Route to αγ-Dichlorohydrin; Ind. Eng. Chem. Res. 46 (2007) 6456-6465.
[23] E. Santacesaria, M. Di Serio, R. Tesser, (Eurochem Engineering srl). Process for monochlorohydrins production from glycerol and hydrochloric acid. PCT Patent WO 132770 A1, 2008.

## Claims

1. Process for the production of α,γ-dichlorohydrin in which glycerol is put in contact with hydrochloric acid in the presence of one or more acyl chloride mono, bi or poly-functional, as catalyst.

2. Process according to claim 1, in which the acyl chloride catalyst has the following general formula (I): 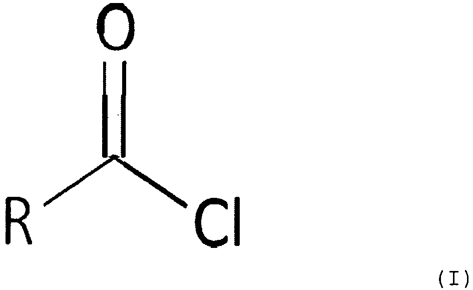 wherein R is an alkyl chain of 2 - 10 carbon atoms which can be linear, branched or cyclic and can contain one or more group R', equal or different chosen between: an aliphatic group linear, branched or cyclic C₁-C₁₀, an aromatic group C₁-C₁₀, an acyl chloride group.

3. Process according to the claims 1 and 2 in which the acyl chloride is one of the followings or their mixtures: acetyl chloride, propanoyl chloride, esandioil di-chloride, butandioil di-chloride, propandioil di-chloride, phenylacetyl chloride

4. Process according to anyone of the claims 1-3 in which hydrochloric acid is dissolved in water or other solvents but preferably is gaseous and anhydrous.

5. Process according to anyone of the claims 1-4, in which glycerol crude or refined comes from plants for the production of biodiesel.

6. Process according to anyone of the claims 1-5, performed in the presence of inorganic salts as alkaline chlorides, preferably sodium or potassium chlorides in a concentration range 1-20% b.w. more preferably in the range 5-10% b.w.

7. Process according to anyone of the claims 1-6, in which the reagents hydrochloric acid and glycerol are fed continuously into the reactor.

8. Process according to anyone of the claims 1-7, in which the reaction is performed at a temperature falling in the range 80 to 150 °C, more preferably in the range 100 to 175°C.

9. Process according to anyone of the claims 1-8, in which the reaction is performer in a range of pressure between 0,5 and 50 bars, more preferably between 5 and 20 bars.

10. Employment of acyl chlorides as catalysts in the reaction between glycerol and hydrochloric acid in the production of chlorohydrins and in particular α,γ-dichlorohydrin.

## Patentansprüche

1. Verfahren zur Herstellung von α, γ-Dichlorhydrin, bei dem Glycerin in Gegenwart von einem oder mehreren Carbonsäurechloriden, mono-, bi- oder polyfunktionell, als Katalysator in Kontakt mit Salzsäure gebracht wird.

2. Verfahren nach Anspruch 1, bei dem der Carbonsäurechlorid-Katalysator die folgende allgemeine Formel (I) aufweist: 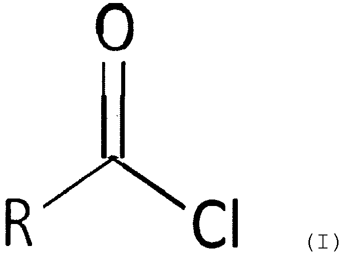 wobei R eine Alkylkette aus 2 bis 10 Kohlenstoffatomen ist, welche linear, verzweigt oder cyclisch sein kann, und eine oder mehrere Gruppen R' enthalten kann, die gleich oder unterschiedlich sein können und ausgewählt sind zwischen: einer aliphatischen Gruppe, linear, verzweigt oder cyclisch mit C₁-C₁₀; einer aromatischen Gruppe mit C₁-C₁₀; einer Carbonsäurechlorid-Gruppe.

3. Verfahren nach Anspruch 1 und 2, bei dem das Carbonsäurechlorid eines der folgenden oder Mischungen daraus ist: Acetylchlorid, Propanoylchlorid, Hexandioil-Dichlorid, Butandioil-Dichlorid, Propandioil-Dichlorid, Phenylacetyl-Chlorid.

4. Verfahren nach einem der Ansprüche 1 bis 3, bei dem Chlorwasserstoffsäure in Wasser oder anderen Lösungsmitteln gelöst ist, aber vorzugsweise gasförmig und anhydrisch ist.

5. Verfahren nach einem der Ansprüche 1 bis 4, bei dem Glycerin unverarbeitet oder verarbeitet von Pflanzen für die Herstellung von Biodiesel kommt.

6. Verfahren nach einem der Ansprüche 1 bis 5, durchgeführt in der Anwesenheit von anorganischen Salzen wie Alkalichloriden, vorzugsweise Natrium- oder Kaliumchloride in einem Konzentrationsbereich von 1 bis 20 % Gewichtsanteil, mehr bevorzugt im Bereich von 5 bis 10 % Gewichtsanteil.

7. Verfahren nach einem der Ansprüche 1 bis 6, bei dem die Reagenzien Salzsäure und Glycerin kontinuierlich in den Reaktor zugeführt werden.

8. Verfahren nach einem der Ansprüche 1 bis 7, bei dem die Reaktion bei einer in den Bereich von 80 bis 150 °C fallenden Temperatur durchgeführt wird, mehr bevorzugt im Bereich von 100 °C bis 175 °C.

9. Verfahren nach einem der Ansprüche 1 bis 8, bei dem die Reaktion in einem Druckbereich zwischen 0,5 und 50 bar durchgeführt wird, mehr bevorzugt zwischen 5 und 20 bar.

10. Verwendung von Carbonsäurechloriden als Katalysatoren in der Reaktion zwischen Glycerin und Salzsäure bei der Herstellung von Chlorhydrinen und insbesondere α, γ-Dichlorhydrin.

## Revendications

1. Procédé de production de α,γ-dichlorhydrine dans lequel du glycérol est mis en contact avec de l'acide chlorhydrique en présence d'un ou plusieurs chlorures d'acyle mono-, bi- ou polyfonctionnels, comme catalyseur.

2. Procédé selon la revendication 1, dans lequel le catalyseur de chlorure d'acyle a la formule générale (I) suivante : 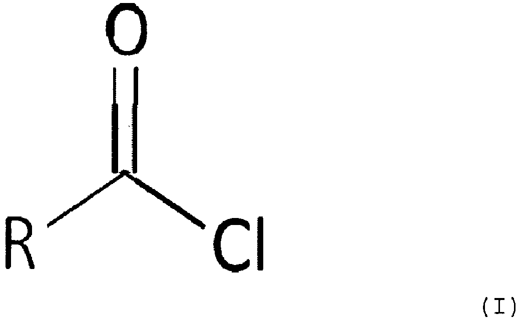 dans lequel R est une chaîne alkyle de 2 - 10 atomes de carbone qui peut être linéaire, ramifiée ou cyclique et peut contenir un ou plusieurs groupes R', identiques ou différents, choisis parmi : un groupe aliphatique linéaire, ramifié ou cyclique C₁-C₁₀, un groupe aromatique C₁-C₁₀, un groupe de chlorure d'acyle.

3. Procédé selon les revendications 1 et 2, dans lequel le chlorure d'acyle est un des éléments suivants ou de leurs mélanges : chlorure d'acétyle, chlorure de propanoyle, di-chlorure d'hexanediol, di-chlorure de butanediol, di-chlorure de propanediol, chlorure de phénylacétyle.

4. Procédé selon l'une quelconque des revendications 1 à 3, dans lequel de l'acide chlorhydrique est dissout dans l'eau ou d'autres solvants mais est de préférence gazeux et anhydre.

5. Procédé selon l'une quelconque des revendications 1 à 4, dans lequel du glycérol brut ou raffiné provient d'installations pour la production de biodiesel.

6. Procédé selon l'une quelconque des revendications 1 à 5, effectué en présence de sels inorganiques tels que des chlorures alcalins, de préférence des chlorures de sodium ou de potassium dans une plage de concentration de 1-20% en poids, de préférence dans la plage de 5-10% en poids.

7. Procédé selon l'une quelconque des revendications 1 à 6, dans lequel les réactifs, l'acide hydrochlorique et le glycérol, sont alimentés en continu dans le réacteur.

8. Procédé selon l'une quelconque des revendications 1 à 7, dans lequel la réaction est effectuée à une température située dans la plage de 80 à 150°C, de préférence dans la plage de 100 à 175°C.

9. Procédé selon l'une quelconque des revendications 1 à 8, dans lequel la réaction est effectuée dans une plage de pression entre 0,5 et 50 bar, de préférence entre 5 et 20 bar.

10. Emploi de chlorures d'acyle comme catalyseurs dans la réaction entre du glycérol et de l'acide hydrochlorique dans la production de chlorhydrines et en particulier de α,γ-dichlorhydrine.
